# EUROPEAN PATENT SPECIFICATION

(11) **EP 4 190 250 B1**
(45) Date of publication and mention of the grant of the patent: **16.07.2025**
(21) Application number: 21853944.3
(22) Date of filing: 03.08.2021
(51) Int. Cl.: A61B 17/11, A61B 17/068, A61B 17/00, H02J 7/00, H02J 9/00

(54) **ELECTRIC STAPLER, AND LOW-POWER-CONSUMING CONTROL METHOD AND DEVICE FOR HANDLE THEREOF, AND STORAGE MEDIUM**
ELEKTRISCHES KLAMMERGERÄT UND STEUERUNGSVERFAHREN MIT GERINGEM ENERGIEVERBRAUCH SOWIE VORRICHTUNG FÜR GRIFF DAFÜR UND SPEICHERMEDIUM
AGRAFEUSE ÉLECTRIQUE, ET PROCÉDÉ DE COMMANDE À FAIBLE CONSOMMATION D'ÉNERGIE ET DISPOSITIF DE POIGNÉE, ET SUPPORT DE STOCKAGE

(30) Priority: 04.08.2020 CN 202010773950
(43) Date of publication of application: 07.06.2023
(73) Proprietor: Intocare Medical Technology (Suzhou) Co., Ltd., Suzhou, Jiangsu 215000 (CN)
(72) Inventor: SHEN, Haiming, Suzhou, Jiangsu 215000 (CN)
(74) Representative: Gulde & Partner
(86) International application number: PCT/CN2021/110313
(87) International publication number: WO 2022/028415

(56) References cited:
- EP-A1- 3 151 308
- CN-A- 101 056 180
- CN-A- 105 324 084
- CN-A- 108 472 069
- CN-A- 108 697 456
- CN-A- 110 799 111
- CN-A- 110 799 117
- CN-A- 110 831 509
- US-A1- 2016 249 927
- US-A1- 2017 202 596
- US-A1- 2018 368 822

## Description

### CROSS-REFERENCE OF RELATED APPLICATION

This application is based on and claims the priority of Chinese Patent Application No.202010773950.3 filed on August 4, 2020 and entitled "electric stapler, and low-power-consuming control method and device for handle thereof, and storage medium".

### TECHNICAL FIELD

The disclosure relates to the technical field of medical apparatus, in particular to a low-power-consuming control method for a handle of an electric stapler, a computer readable storage medium, an electric stapler and a low-power-consuming control device for the handle of the electric stapler.

### BACKGROUND

In electric tools, battery capacity is the key to determine whether the electric tool can successfully complete a certain task. The task may be successfully completed by using a high-capacity battery pack, but the volume, weight and storage space of the battery pack has to be increased.

For example, an electric stapler is usually powered by a lithium battery, and the capacity of the lithium battery is the key to determine whether a surgical operation can be successfully completed. A high-capacity battery pack may be used to complete the surgical operation, but the volume of the battery pack has to be increased, which in turn leads to the increase in the volume and weight of the whole electric stapler, so that the user needs to spend a lot of physical strength to complete the surgical operation, and the user experience is poor. At the same time, the storage of the electric stapler becomes difficult because of the limitation of the storage space. US2018368822 (A1) provides an apparatus, and the apparatus includes a handle assembly and a battery pack. The handle assembly includes a housing and a first control circuit located within the housing. The battery pack assembly includes a power supply and a second control circuit. The power supply is configured to selectively transition from a first state to a second state. The power supply is configured to energize the first control circuit of the handle assembly in the first state. The power supply is configured to not energize the first control circuit of the handle assembly in the second state. The second control circuit is configured to transition the power supply between the first state and the second state. EP3151308A1 discloses a surgical instrument. The surgical instrument includes a shaft assembly, a handle assembly including a battery dock, and a battery unit. The battery unit is configured to be received by the battery dock such that the battery unit is in electrical communication with at least one of the shaft assembly or the handle assembly. The battery unit includes a casing, an anode contact and a cathode contact within the casing, and a discharge drain. The discharge drain includes a controller, a switch element, and a resistor element operatively connected between the cathode contact and the anode contact. The controller is configured to selectively direct the switch element to close such that the resistor element is in electrical communication with the anode contact and the cathode contact. Thereby, the resistor element is configured to drain remaining electrical power from the at least one battery connected to the anode contact and the cathode contact.

### SUMMARY

### (1) Technical problems to be solved

In view of the above shortcomings and disadvantages of the prior art, it is an object of the present disclosure to provide an electric stapler, a low-power-consuming control method and device for a handle of the electric stapler, and a storage medium, which solves the technical problems that the volume, weight and storage space of the electric stapler has to be increased caused by the electric stapler being powered by a high-capacity battery pack to ensure that the electric stapler can work for a long time.

### (2) Technical solutions

The object is achieved by the features of the respective independent claims. Further embodiments are defined in the corresponding dependent claims. Even though the description refers to embodiments or to the invention, it is to be understood that the invention is defined by the claims and embodiments of the invention are those comprising at least all the features of one of the independent claims

According to the electric stapler provided by the embodiments of the present disclosure, the low-power-consuming control device for the handle of the electric stapler is provided, the power consumption of the electric handle reaches the lowest level, and the power consumption occurs when the electric handle is not in use is reduced, so that under the condition that the capacity of the battery pack is unchanged, as much power is remained as possible to ensure that the electric handle is capable of working for a long time.

### (3) Beneficial effects

The beneficial effects of the disclosure is as follows: in the electric stapler, the low-power-consuming control method and device for the handle of the electric stapler, and the storage medium provided by the disclosure, in the case that the electric handle is not in use, the driving power supply network and the Hall power supply network of the electric stapler are controlled to stop power supply output, and the handle controller is triggered to enter the dormant state, so that the electric handle enters the low-power-consuming mode. Thus, comparing the prior art, the power consumption occurs when the electric handle is not in use is reduced, so that under the condition that the capacity of the battery pack is unchanged, as much power is remained as possible to ensure that the electric handle is capable of working for a long time.

### BRIEF DESCRIPTION OF THE DRAWINGS

In order to clearly illustrate the technical solutions of embodiments of the disclosure, the drawings of the embodiments of the disclosure will be briefly described in the following. It is obvious that the described drawings are only related to some embodiments of the disclosure and thus do not limit the scope of the disclosure.
Fig. 1 is a flowchart of a low-power-consuming control method for a handle of an electric stapler according to one embodiment of the present disclosure;
Fig. 2 is a circuit diagram of a driving power supply network according to one embodiment of the present disclosure;
Fig. 3 is a circuit diagram of a Hall power supply network according to one embodiment of the present disclosure;
Fig. 4 is a flowchart of a low-power-consuming control method for a handle of an electric stapler according to another embodiment of the present disclosure;
Fig. 5 is a structural schematic diagram of a low-power-consuming control device for a handle of an electric stapler according to one embodiment of the present disclosure.

### DETAILED DESCRIPTION

In order to better explain the present disclosure and facilitate understanding of the disclosure, the following detailed description of the present disclosure will be made with reference to the drawings.

According to an electric stapler, a low-power-consuming control method and device for a handle of the electric stapler, and a storage medium provided by embodiments of the disclosure, the timing starts upon installing a battery pack into a battery compartment,, and at the same time it detects whether the electric handle receives an operation instruction or not and controls an adapter to identify a working head; if a timing duration reaches a first preset time, the electric handle does not receive the operation instruction and the adapter does not identify the working head, then a driving power supply network and a Hall power supply network of the electric stapler are controlled to stop power supply output and a handle controller is triggered to enter a dormant state, so that the electric handle enters a low-power-consuming mode and a power consumption of the electric handle reaches a lowest level. Thus, a power consumption occurs when the electric stapler is not in use is reduced, so that under the condition that the capacity of the battery pack is unchanged, as much power is remained as possible to ensure that the electric stapler is capable of working for a long time, which effectively solves the technical problems that the volume, weight and storage space of the electric stapler has to be increased caused by the electric stapler being powered by a high-capacity battery pack to ensure that the electric stapler can work for a long time.

In order to better understand the above technical solutions, the exemplary embodiments of the present disclosure will be described in more detail below with reference to the drawings. Although exemplary embodiments of the present disclosure are shown in the drawings, it should be understood that the present disclosure can be implemented in various forms and should not be limited to the embodiments set forth herein. On the contrary, these embodiments are provided to enable a clearer and more thorough understanding of the disclosure, and to fully convey the scope of the disclosure to those skilled in the art.

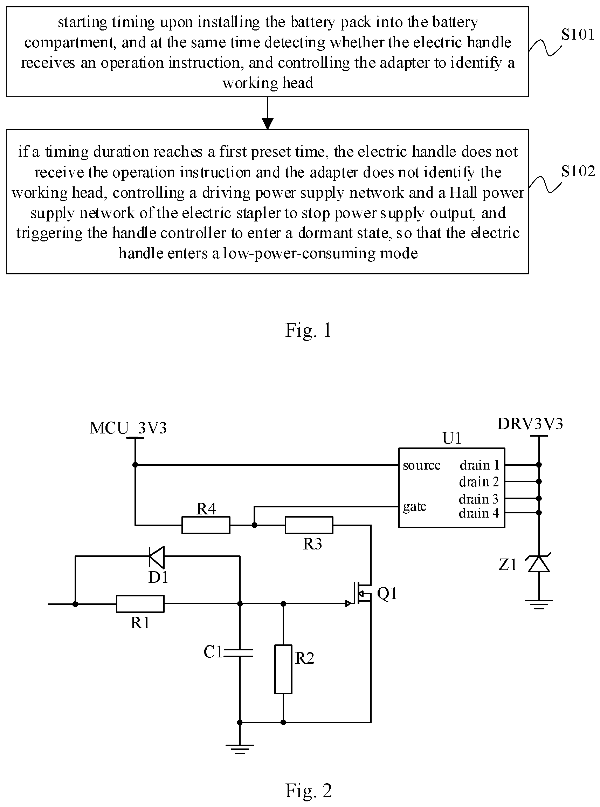

Fig. 1 is a flowchart of a low-power-consuming control method for a handle of an electric stapler according to one embodiment of the present disclosure. In the disclosure, the electric stapler includes an electric handle and an adapter, and a battery compartment for installing a battery pack is provided in the electric handle. Referring to Fig. 1, the low-power-consuming control method for the handle of the electric stapler includes the following steps:
S101, starting timing upon installing the battery pack into the battery compartment, and at the same time detecting whether the electric handle receives an operation instruction, and controlling the adapter to identify a working head.

Specifically, upon installing the battery pack into the battery compartment, a handle controller of the electric handle controls a timer and the like to start timing. At the same time, it is detected in real time whether the electric handle receives the operation instruction sent by a user through a control key and the like, and the adapter is controlled to identify the working head. That is, upon installing the battery pack into the battery compartment, timing is started, and it is judged in real time whether the electric handle is to be used or not.

S102, if a timing duration reaches a first preset time, the electric handle does not receive the operation instruction and the adapter does not identify the working head, controlling a driving power supply network and a Hall power supply network of the electric stapler to stop power supply output, and triggering the handle controller to enter a dormant state, so that the electric handle enters a low-power-consuming mode. The first preset time is set according to the actual situation, and optionally, the first preset time is 30-50 seconds.

Specifically, if the timing duration reaches the first preset time (such as 40 seconds), and if the electric handle does not receive the operation instruction sent by the user through the control key and the like and the adapter does not identify the working head, it means that the electric handle will not be used at present. At this time, the handle controller controls the driving power supply network and the Hall power supply network of the electric stapler to stop power supply output, and automatically enters the dormant state, so that the electric handle enters the low-power-consuming mode. That is to say, in the case that the electric handle is not used, the handle controller cut off the power supply to other elements to eliminate the power consumption caused by other elements, and then the handle controller automatically enters the low-power-consuming mode, such as keeping only a clock function and an interrupt function, so as to minimize the power consumption of the electric handle, and thus under the condition that the capacity of the battery pack is unchanged, as much power is remained as possible to ensure that the electric handle is capable of working for a long time.

According to the low-power-consuming control method for the handle of the electric stapler in the embodiments of the present disclosure, in the case that the battery pack is installed in the battery compartment of the electric handle but the electric handle is not used, the driving power supply network and the Hall power supply network of the electric stapler are controlled to stop power supply output, and the handle controller is triggered to enter the dormant state, so that the electric handle enters the low-power-consuming mode and the power consumption of the electric handle reaches the lowest level. Thus, the power consumption occurs when the electric handle is not in use is reduced, so that under the condition that the capacity of the battery pack is unchanged, as much power is remained as possible to ensure that the electric handle is capable of working for a long time.

According to one embodiment of the present disclosure, the handle controller outputs a low-level control signal to the driving power supply network and the Hall power supply network, respectively, so as to control the driving power supply network and the Hall power supply network to stop power supply output.

Specifically, Fig. 2 is a circuit diagram of a driving power supply network according to one embodiment of the present disclosure. Referring to Fig. 2, the driving power supply network includes: a first switch tube Q1, a first resistor R1, a first diode D1, a second resistor R2, a first capacitor C1, a third resistor R3, a fourth resistor R4, a first switch chip U1, and a first voltage stabilizing tube Z1, wherein the second resistor R2 and the first capacitor C1 are connected in parallel, the third resistor R3 and the fourth resistor R4 are connected in series. A control terminal of the first switch tube Q1 is connected to the handle controller through the first resistor R1, and a first terminal of the first switch tube Q1 is grounded. The first diode D1 is connected in parallel with the first resistor R1, and an anode of the first diode D1 is connected with the control terminal of the first switch Q1; the second resistor R2 and the first capacitor C1 are connected in parallel between the control terminal of the first switch tube Q1 and the ground; one terminal of the third resistor R3 is connected to a second terminal of the first switch tube Q1, and the other terminal of the third resistor R3 is connected to one terminal of the fourth resistor R4 and has a first node therebetween, and the other terminal of the fourth resistor R4 is connected to a first power supply such as MCU _3V3; a power input terminal of the first switch chip U1 is connected with the first power supply, a grounding terminal of the first switch chip U1 is connected with the first node, and a plurality of power output terminals of the first switch chip U1 are used as power output terminals of the driving power supply network to provide driving power such as DRV3V3 to a back-end load; a cathode of the first voltage stabilizing tube Z1 is connected to the plurality of power output terminals of the first switch chip U1, and an anode of the first voltage stabilizing tube Z1 is grounded.

In the case that the handle controller outputs the low-level control signal to the driving power supply network, the control terminal of the first switch tube Q1 in the driving power supply network receives the low-level signal, and both the first switch tube Q1 and the first switch chip U1 are in the off state. At this time, the plurality of power output terminals of the first switch chip U1 have no voltage output, that is, the DRV3V3 has no electricity, and corresponding elements connected to the driving power supply network, such as a motor driving chip, a memory chip, an operational amplifier and the like, are not supplied with power, and the power consumption of the elements is zero. Therefore, the power consumption caused by the elements connected to the driving power supply network can be eliminated.

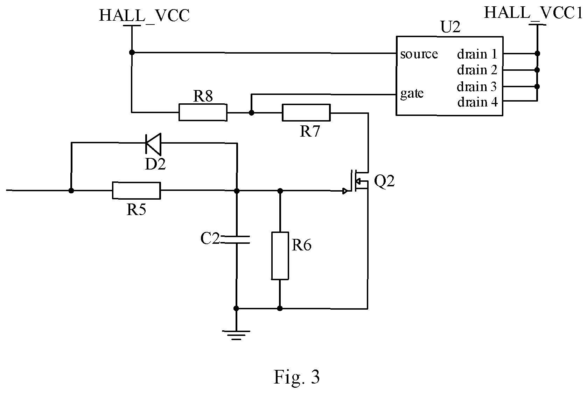

Fig. 3 is a circuit diagram of a Hall power supply network according to one embodiment of the present disclosure. Referring to Fig. 3, the Hall power supply network includes: a second switch tube Q2, a fifth resistor R5, a second diode D2, a sixth resistor R6, a second capacitor C2, a seventh resistor R7, an eighth resistor R8, and a second switch chip U2, wherein the sixth resistor R6 and the second capacitor C2 are connected in parallel, the seventh resistor R7 and the eighth resistor R8 are connected in series. A control terminal of the second switch tube Q2 is connected to the handle controller through the fifth resistor R5, and a first terminal of the second switch tube Q2 is grounded; the second diode D2 is connected in parallel with the fifth resistor R5, and an anode of the second diode D2 is connected with the control terminal of the second switch tube Q2; the sixth resistor R6 and the second capacitor C2 are connected in parallel between the control terminal of the second switch Q2 and the ground; one terminal of the seventh resistor R7 is connected to a second terminal of the second switch tube Q2, the other terminal of the seventh resistor R7 is connected to one terminal of the eighth resistor R8 and has a second node therebetween, and the other terminal of the eighth resistor R8 is connected to a second power supply such as HALL_VCC; a power input terminal of the second switch chip U2 is connected to the second power supply, a grounding terminal of the second switch chip U2 is connected to the second node, and a plurality of power output terminals of the second switch chip U2 are used as power output terminals of the Hall power supply network to provide driving power such as HALL_VCC1 to the back-end load.

In the case that the handle controller outputs the low-level control signal to the Hall power supply network, the control terminal of the second switch tube Q2 in the Hall power supply network receives the low-level signal, and both the second switch tube Q2 and the second switch chip U2 are in the off state. At this time, the plurality of power output terminals of the second switch chip U2 has no voltage output, that is, HALL_VCC1 has no electricity, and corresponding Hall elements connected to the Hall power supply network, such as a Hall sensor, are not supplied with power, and the power consumption of the elements is zero. Therefore, the power consumption caused by elements connected to the Hall power supply network can be eliminated.

According to one embodiment of the present disclosure, if the electric handle is in the low-power-consuming mode, and if the electric handle receives the operation instruction or the adapter identifies the working head, then the electric handle is controlled to exit the low-power-consuming mode and enter a normal working mode.

Specifically, if the electric handle is in the low-power-consuming mode, and if the electric handle receives the operation instruction sent by the user through the control key and the like or the adapter identifies the working head, then the handle controller receives the corresponding signal in an interrupted way, and the handle controller controls the electric handle to exit the low-power-consuming mode and enter the normal working mode. That is, in the case that the electric handle is used, the electric handle exits the low-power-consuming mode and enters the normal working mode.

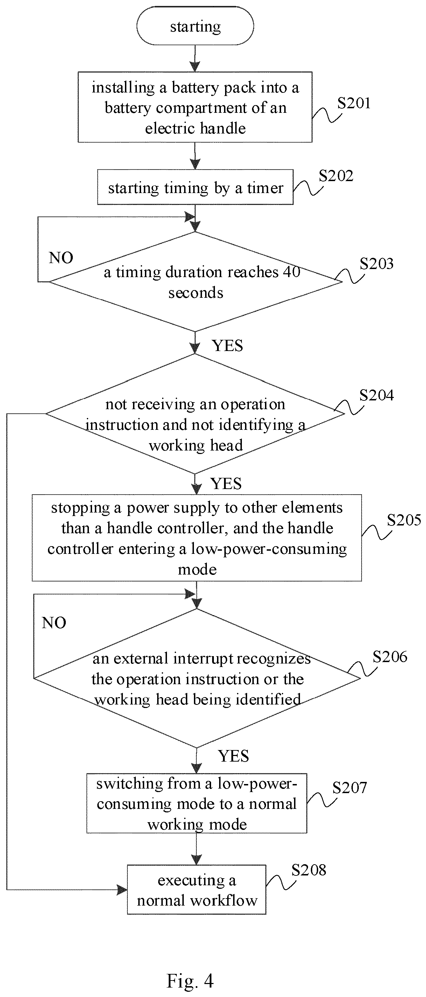

Further, as a specific example, referring to Fig. 4, the low-power-consuming control method for the handle of the electric stapler includes the following steps:
S201, installing the battery pack into the battery compartment of the electric handle.
S202, starting timing by the timer.
S203, judging whether the timing duration reaches 40 seconds. If yes, step S204 is executed; otherwise, it is continued to perform this step.
S204, judging whether the operation instruction is received and identifying the working head. If the operation instruction is not received and the working head is not identified, step S205 is executed; otherwise, it is continued to perform this step.
S205, stopping the power supply to other elements than the handle controller, and the handle controller entering the low-power-consuming mode. For example, the power supply to other elements than the handle controller MCU is stopped, and at the same time, the handle controller MCU enters its own low-power-consuming mode LPM3, only the auxiliary clock function and the interrupt function are kept to minimize the power consumption.
S206, judging whether an external interrupt recognizes the operation instruction or the working head being identified, that is, whether the external interrupt of the handle controller MCU recognizes the operation key of the electric handle being pressed or the working head being connected. If yes, step S207 is executed; otherwise, it is continued to perform this step.
S207, switching from the low-power-consuming mode to the normal working mode.
S208, executing a normal workflow.

In summary, according to the low-power-consuming control method for the handle of the electric stapler provided by the embodiments of the disclosure, in the case that the electric handle is not in use, the driving power supply network and the Hall power supply network of the electric stapler are controlled to stop power supply output, and the handle controller is triggered to enter the dormant state, so that the electric handle enters the low-power-consuming mode and the power consumption of the electric handle reaches the lowest level. Thus, the power consumption occurs when the electric handle is not in use is reduced, so that under the condition that the capacity of the battery pack is unchanged, as much power is remained as possible to ensure that the electric handle is capable of working for a long time, and thus the surgical operation lasting for a long time and alternately using multiple devices can be successfully completed.

In addition, the embodiments of the present disclosure further provide a computer readable storage medium in which a low-power-consuming control program for the handle of the electric stapler is stored, and when the low-power-consuming control program for the handle is executed by a processor, the above-mentioned low-power-consuming control method for the handle of the electric stapler is realized.

According to the computer readable storage medium provided by the embodiments of the present disclosure, by using the low-power-consuming control method for the handle of the electric stapler, the power consumption of the electric handle reaches the lowest level, and the power consumption occurs when the electric handle is not in use is reduced, so that under the condition that the capacity of the battery pack is unchanged, as much power is remained as possible to ensure that the electric handle is capable of working for a long time.

In addition, the embodiments of the disclosure further provide an electric stapler, the electric stapler comprises an electric handle and an adapter, a battery compartment for installing a battery pack is provided in the electric handle, and the electric handle further comprises a memory, a processor and a low-power-consuming control program for the handle of the electric stapler which is stored in the memory and executed by the processor; and in the case that the processor executes the low-power-consuming control program for the handle, the low-power-consuming control method for the handle of the electric stapler is realized.

According to the electric stapler provided by the embodiments of the present disclosure, by using the low-power-consuming control method for the handle of the electric stapler is realized, the power consumption of the electric handle reaches the lowest level, and the power consumption occurs when the electric handle is not in use is reduced, so that under the condition that the capacity of the battery pack is unchanged, as much power is remained as possible to ensure that the electric handle is capable of working for a long time.

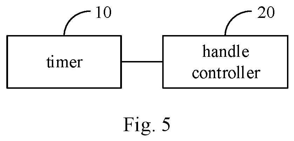

Fig. 5 is a structural schematic diagram of a low-power-consuming control device for a handle of an electric stapler according to one embodiment of the present disclosure. The electric stapler includes an electric handle and an adapter, and a battery compartment for installing a battery pack is provided in the electric handle. Referring to Fig. 5, the low-power-consuming control device for the handle of the electric stapler includes a timer 10 and a handle controller 20.

The handle controller 20 is configured to trigger the timer 10 to start timing upon installing the battery pack into the battery compartment, and is further configured to detect whether the electric handle receives the operation instruction, and control the adapter to identify the working head. The handle controller 20 is further configured to control the driving power supply network and the Hall power supply network of the electric stapler to stop power supply output and enter the dormant state, if a timing duration of the timer 10 reaches the first preset time, the electric handle does not receive the operation instruction, and the adapter does not identify the working head, so that the electric handle enters the low-power-consuming mode.

According to one embodiment of the present disclosure, the handle controller 20 is further configured to output a low-level control signal to the driving power supply network and the Hall power supply network, respectively, to control the driving power supply network and the Hall power supply network to stop power supply output.

According to one embodiment of the present disclosure, the handle controller 20 is further configured to control the electric handle to exit the low-power-consuming mode and enter the normal working mode, if the electric handle receives the operation instruction or the adapter identifies the working head in the case that the electric handle is in the low-power-consuming mode.

It should be noted that, for the low-power-consuming control device for the handle of the electric stapler in the disclosure, the description of the low-power-consuming control method for the handle of the electric stapler in the disclosure may be referred to, and the details will not be repeated here.

According to the low-power-consuming control device for the handle of the electric stapler provided by the embodiments of the present disclosure, the timing starts upon installing the battery pack into the battery compartment, and at the same time it detects whether the electric handle receives the operation instruction or not and controls the adapter to identify the working head; if the timing duration reaches the first preset time, the electric handle does not receive the operation instruction and the adapter does not identify the working head, then the driving power supply network and the Hall power supply network of the electric stapler are controlled to stop power supply output and the handle controller is triggered to enter the dormant state, so that the electric handle enters the low-power-consuming mode and the power consumption of the electric handle reaches the lowest level. Thus, the power consumption occurs when the electric stapler is not in use is reduced, so that under the condition that the capacity of the battery pack is unchanged, as much power is remained as possible to ensure that the electric stapler is capable of working for a long time.

In addition, the embodiments of the present disclosure provide an electric stapler, and the electric stapler comprises the above mentioned low-power-consuming control device for the handle of the electric stapler.

According to the electric stapler provided by the embodiments of the present disclosure, by using the low-power-consuming control device for the handle of the electric stapler, the power consumption of the electric handle reaches the lowest level, and the power consumption occurs when the electric handle is not in use is reduced, so that under the condition that the capacity of the battery pack is unchanged, as much power is remained as possible to ensure that the electric handle is capable of working for a long time.

In the description of the disclosure, it should be understood that the terms "first" and "second" are only used for descriptive purpose, and cannot be understood as indicating or implying relative importance or implicitly indicating the total number of the indicated technical features. Therefore, the features defined by "first" and "second" may include one or more explicitly or implicitly. In the description of the disclosure, "a plurality of" means two or more, unless otherwise specifically defined.

In the disclosure, unless otherwise specified and limited, terms such as "install", "communicate", "connect" and "fix" should be understood broadly; for example, it may be a fixed connection, a detachable connection or an integral structure; it may be a mechanical connection or an electrical connection; it may be a direct connection or an indirect connection through an intermediate medium; it may be the internal communication of two elements or the mutual interaction relationship between two elements. For an ordinary skilled in the art, the specific meanings of the above terms in the disclosure may be understood according to specific situations.

In the disclosure, unless otherwise specified and limited, the first feature being "above" or "below" the second feature may be the direct contact between the first feature and the second feature, or the indirect contact between the first feature and the second feature through an intermediate medium. Furthermore, the first feature being "above", "over" and "on" the second feature may be the first feature being directly above or obliquely above the second feature, or simply means that a horizontal height of the first feature is higher than that of the second feature. The first feature being "below", "under" and "beneath" the second feature may be the first feature being directly below or obliquely below the second feature, simply means that the horizontal height of the first feature is lower than that of the second feature.

In the description of the specification, the terms "one embodiment", "some embodiments", "embodiments", "examples", "specific examples" or "some examples" mean that the specific features, structures, materials or characteristics described in connection with the embodiment or example are included in at least one embodiment or example of the disclosure. In the description, the schematic expressions of the above terms do not necessarily refer to the same embodiment or example. Furthermore, the specific described features, structures, materials or characteristics may be combined in any one or more embodiments or examples in a suitable manner. In addition, those skilled in the art can combine different embodiments or examples and features of different embodiments or examples described in the specification without conflicting with each other.

Although the embodiments of the present disclosure have been illustrated and described above, it should be understood that the above embodiments are illustrative and should not be construed as limitations of the present disclosure, and those skilled in the art can make changes, modifications, substitutions and variations to the above embodiments within the scope of the present disclosure.

## Claims

1. A low-power-consuming control method for a handle of an electric stapler, the electric stapler comprising an electric handle and an adapter, the electric handle comprising a battery compartment configured for installing a battery pack, and the low-power-consuming control method for the handle comprising:
starting timing upon installing the battery pack into the battery compartment, and at the same time detecting whether the electric handle receives an operation instruction and controlling the adapter to identify a working head (S101);
if a timing duration reaches a first preset time, the electric handle does not receive an operation instruction and the adapter does not identify a working head, controlling a driving power supply network and a Hall power supply network of the electric stapler to stop power supply output, and triggering a handle controller (20) to enter a dormant state, so that the electric handle enters a low-power-consuming mode (S102),
wherein the Hall power supply network comprises: a second switch tube (Q2), a fifth resistor (R5), a second diode (D2), a sixth resistor (R6), a second capacitor (C2), a seventh resistor (R7), an eighth resistor (R8), and a second switch chip (U2), wherein the sixth resistor (R6) and the second capacitor (C2) are connected in parallel, the seventh resistor (R7) and the eighth resistor (R8) are connected in series,
wherein controlling a driving power supply network and a Hall power supply network of the electric stapler to stop power supply output, comprises:
outputting by the handle controller (20) a low-level control signal to the driving power supply network and the Hall power supply network, respectively,
wherein a control terminal of the second switch tube (Q2) is configured to receive the low-level control signal, and both the second switch tube (Q2) and the second switch chip (U2) are in an off state.

2. The low-power-consuming control method for the handle of the electric stapler according to claim 1, wherein the electric handle is in the low-power-consuming mode and the low-power-consuming control method further comprising:
if the electric handle receives the operation instruction or the adapter identifies the working head, controlling the electric handle to exit the low-power-consuming mode and enter a normal working mode.

3. The low-power-consuming control method for the handle of the electric stapler according to claim 1, wherein the first preset time is 30-50 seconds.

4. A computer readable storage medium, in which a low-power-consuming control program for a handle of an electric stapler is stored,
wherein the low-power-consuming control method for the handle of the electric stapler according to any one of claims 1-3 is realized by executing the low-power-consuming control program for the handle.

5. An electric stapler, comprising:
an electric handle, comprising a battery compartment, wherein the battery compartment is configured for installing a battery pack; and
an adapter,
wherein the electric handle further comprises:
a memory, configured for storing a low-power-consuming control program for the handle of the electric stapler; and
a processor, configured for executing the low-power-consuming control program for the handle, such that the low-power-consuming control method for the handle of the electric stapler according to any one of claims 1-4 is realized.

6. A low-power-consuming control device for a handle of an electric stapler, wherein the electric stapler comprises an electric handle and an adapter, the electric handle comprises a battery compartment configured for installing a battery pack, the low-power-consuming control device for the handle comprising:
a timer (10); and
a handle controller (20), wherein:
the handle controller (20) is configured to detect whether the electric handle receives an operation instruction, and control the adapter to identify a working head;
**characterized in that**,
the handle controller (20) is configured to trigger the timer (10) to start timing upon installing the battery pack into the battery compartment,
the handle controller (20) is further configured to control a driving power supply network and a Hall power supply network of the electric stapler to stop power supply output and enter a dormant state, if a timing duration of the timer (10) reaches a first preset time, the electric handle does not receive the operation instruction, and the adapter does not identify the working head, so that the electric handle enters a low-power-consuming mode,
the handle controller (20) is further configured to output a low-level control signal to the driving power supply network and the Hall power supply network, respectively, so as to control the driving power supply network and the Hall power supply network to stop power supply output,
the Hall power supply network comprises: a second switch tube (Q2), a fifth resistor (R5), a second diode (D2), a sixth resistor (R6), a second capacitor (C2), a seventh resistor (R7), an eighth resistor (R8), and a second switch chip (U2), wherein the sixth resistor (R6) and the second capacitor (C2) are connected in parallel, the seventh resistor (R7) and the eighth resistor (R8) are connected in series,
a control terminal of the second switch tube (Q2) is configured to receive the low-level control signal, and both the second switch tube (Q2) and the second switch chip (U2) are in an off state.

7. The low-power-consuming control device for the handle of the electric stapler according to claim 6, wherein the driving power supply network comprising:
a first switch tube (Q1), a first resistor (R1), a first diode (D1), a second resistor (R2), a first capacitor (C1), a third resistor (R3), a fourth resistor (R4), a first switch chip (U1), and a first voltage stabilizing tube (Z1), wherein the second resistor (R2) and the first capacitor (C1) are connected in parallel, the third resistor (R3) and the fourth resistor (R4) are connected in series.

8. The low-power-consuming control device for the handle of the electric stapler according to claim 7, wherein a control terminal of the first switch tube (Q1) is configured to receive the low-level control signal, and both the first switch tube (Q1) and the first switch chip (U1) are in an off state.

9. The low-power-consuming control device for the handle of the electric stapler according to claim 6, wherein the handle controller (20) is further configured to control the electric handle to exit the low-power-consuming mode and enter a normal working mode, if the electric handle receives the operation instruction or the adapter identifies the working head in the case that the electric handle is in the low-power-consuming mode.

10. An electric stapler, comprising the low-power-consuming control device for the handle of the electric stapler according to any one of claims 6-9.

## Patentansprüche

1. Steuerungsverfahren mit geringem Energieverbrauch für einen Griff eines elektrischen Klammergeräts, wobei das elektrische Klammergerät einen elektrischen Griff und einen Adapter umfasst, wobei der elektrische Griff ein Batteriefach umfasst, das zum Einlegen eines Batteriepacks ausgestaltet ist, und das Steuerungsverfahren mit geringem Energieverbrauch für den Griff Folgendes umfasst:
Starten der Zeitmessung bei Einlegen des Batteriepacks in das Batteriefach und gleichzeitig Erkennen, ob der elektrische Griff eine Betriebsanweisung empfängt, sowie Kontrollieren des Adapters zum Identifizieren eines Arbeitskopfes (S101);
wenn eine Dauer der Zeitmessung eine erste voreingestellte Zeit erreicht, der elektrische Griff keine Betriebsanweisung erhält und der Adapter keinen Arbeitskopf identifiziert, Steuern eines Antriebsenergieversorgungsnetzes und eines Hall-Energieversorgungsnetzes des elektrischen Klammergeräts, sodass die Energieversorgungsausgabe gestoppt wird, und Auslösen einer Griffsteuerung (20) zum Eintreten in einen Ruhezustand, sodass der elektrische Griff in einen Modus mit geringem Energieverbrauch eintritt (S102),
wobei das Hall-Energieversorgungsnetz Folgendes umfasst: eine zweite Schaltröhre (Q2),
einen fünften Widerstand (R5), eine zweite Diode (D2), einen sechsten Widerstand (R6),
einen zweiten Kondensator (C2), einen siebten Widerstand (R7), einen achten Widerstand (R8) und einen zweiten Schalt-Chip (U2), wobei der sechste Widerstand (R6) und der zweite Widerstand (C2) parallel verbunden sind und der siebte Widerstand (R7) und der achte Widerstand (R8) in Reihe verbunden sind,
wobei das Steuern eines Antriebsenergieversorgungsnetzes und eines Hall-Energieversorgungsnetzes des elektrischen Klammergeräts zum Stoppen der Energieversorgungsausgabe Folgendes umfasst:
Ausgeben, durch die Griffsteuerung (20), eines Steuerungssignals für ein geringes Niveau an jeweils das Antriebsenergieversorgungsnetz und das Hall-Energieversorgungsnetz,
wobei ein Steuerungsanschluss der zweiten Schaltröhre (Q2) dazu ausgestaltet ist, das Steuerungssignal für ein geringes Niveau zu empfangen, und sowohl die zweite Schaltröhre (Q2) als auch der zweite Schalt-Chip (U2) in einem Aus-Zustand sind.

2. Steuerungsverfahren mit geringem Energieverbrauch für den Griff des elektrischen Klammergeräts nach Anspruch 1, wobei der elektrische Griff im Modus mit geringem Energieverbrauch ist und das Steuerungsverfahren mit geringem Energieverbrauch ferner Folgendes umfasst:
wenn der elektrische Griff die Betriebsanweisung erhält oder der Adapter den Arbeitskopf identifiziert, Steuern des elektrischen Griffs, sodass er den Modus mit geringem Energieverbrauch verlässt und in einen normalen Arbeitsmodus eintritt.

3. Steuerungsverfahren mit geringem Energieverbrauch für den Griff des elektrischen Klammergeräts nach Anspruch 1, wobei die erste voreingestellte Zeit 30 bis 50 Sekunden beträgt.

4. Rechnerlesbares Speichermedium, in dem ein Steuerungsprogramm mit geringem Energieverbrauch für einen Griff eines elektrischen Klammergeräts gespeichert ist,
wobei das Steuerungsverfahren mit geringem Energieverbrauch für den Griff des elektrischen Klammergeräts nach einem der Ansprüche 1 bis 3 durch Ausführen des Steuerungsverfahrens mit geringem Energieverbrauch für den Griff realisiert wird.

5. Elektrisches Klammergerät, umfassend:
einen elektrischen Griff, umfassend ein Batteriefach, wobei das Batteriefach zum Einlegen eines Batteriepacks ausgestaltet ist; und
einen Adapter,
wobei der elektrische Griff ferner Folgendes umfasst:
einen Speicher, dazu ausgestaltet, ein Programm mit geringerem Energieverbrauch für den Griff des elektrischen Klammergeräts zu speichern; und
einen Prozessor, ausgestaltet zum Ausführen des Steuerungsprogramms mit geringem Energieverbrauch für den Griff, sodass das Steuerungsverfahren mit geringerem Energieverbrauch für den Griff des elektrischen Klammergeräts nach einem der Ansprüche 1 bis 4 realisiert wird.

6. Steuerungsvorrichtung mit geringem Energieverbrauch für einen Griff eines elektrischen Klammergeräts, wobei das elektrische Klammergerät einen elektrischen Griff und einen Adapter umfasst, wobei der elektrische Griff ein Batteriefach umfasst, das zum Einlegen eines Batteriepacks ausgestaltet ist, und die Steuerungsvorrichtung mit geringem Energieverbrauch für den Griff umfasst:
einen Zeitmesser (10); und
eine Griffsteuerung (20), wobei:
die Griffsteuerung (20) dazu ausgestaltet ist, zu erkennen, ob der elektrische Griff eine Betriebsanweisung empfängt, und den Adapter so zu steuern, dass er einen Arbeitskopf identifiziert;
**dadurch gekennzeichnet, dass**
die Griffsteuerung (20) dazu ausgestaltet ist, den Zeitmesser (10) auszulösen, um bei Einlegen des Batteriepacks in das Batteriefach die Zeitmessung zu starten,
die Griffsteuerung (20) ferner dazu ausgestaltet ist, ein Antriebsenergieversorgungsnetz und ein Hall-Energieversorgungsnetz des elektrischen Klammergeräts so zu steuern, dass die Energieversorgungsausgabe gestoppt und in einen Ruhezustand eingetreten wird, wenn eine erste Zeitmessungsdauer des Zeitmessers (10) eine erste voreingestellte Zeit erreicht, der elektrische Griff die Betriebsanweisung nicht erhält und der Adapter den Arbeitskopf nicht identifiziert, sodass der elektrische Griff in einen Modus mit geringem Energieverbrauch eintritt,
die Griffsteuerung (20) ferner dazu ausgestaltet ist, ein Steuerungssignal für ein geringes Niveau an jeweils das Antriebsenergieversorgungsnetz und das Hall-Energieversorgungsnetz auszugeben, um das Antriebsenergieversorgungsnetz und das Hall-Energieversorgungsnetz so zu steuern, dass die Energieversorgungsausgabe gestoppt wird,
wobei das Hall-Energieversorgungsnetz Folgendes umfasst: eine zweite Schaltröhre (Q2),
einen fünften Widerstand (R5), eine zweite Diode (D2), einen sechsten Widerstand (R6),
einen zweiten Kondensator (C2), einen siebten Widerstand (R7), einen achten Widerstand (R8) und einen zweiten Schalt-Chip (U2), wobei der sechste Widerstand (R6) und der zweite Kondensator (C2) parallel verbunden sind und der siebte Widerstand (R7) und der achte Widerstand (R8) in Reihe verbunden sind,
wobei ein Steuerungsanschluss der zweiten Schaltröhre (Q2) dazu ausgestaltet ist, das Steuerungssignal für ein geringes Niveau zu empfangen, und sowohl die zweite Schaltröhre (Q2) als auch der zweite Schalt-Chip (U2) in einem Aus-Zustand sind.

7. Steuerungsverfahren mit geringem Energieverbrauch für den Griff des elektrischen Klammergeräts nach Anspruch 6, wobei das Antriebsenergieversorgungsnetz Folgendes umfasst:
eine erste Schaltröhre (Q1), einen ersten Widerstand (R1), eine erste Diode (D1), einen zweiten Widerstand (R2), einen ersten Kondensator (C1), einen dritten Widerstand (R3), einen vierten Widerstand (R4), einen ersten Schalt-Chip (U1) und eine erste Spannungsstabilisierungsröhre (Z1), wobei der zweite Widerstand (R2) und der erste Kondensator (C1) parallel verbunden sind und der dritte Widerstand (R3) und der vierte Widerstand (R4) in Reihe verbunden sind.

8. Steuerungsvorrichtung mit geringem Energieverbrauch für den Griff des elektrischen Klammergeräts nach Anspruch 7, wobei ein Steuerungsanschluss der ersten Schaltröhre (Q1) dazu ausgestaltet ist, das Steuerungssignal für ein geringes Niveau zu empfangen, und sowohl die erste Schaltröhre (Q1) als auch der erste Schalt-Chip (U1) in einem Aus-Zustand sind.

9. Steuerungsvorrichtung mit geringem Energieverbrauch für den Griff des elektrischen Klammergeräts nach Anspruch 6, wobei die Griffsteuerung (20) ferner dazu ausgestaltet ist, den elektrischen Griff so zu steuern, dass der Modus mit geringem Energieverbrauch verlassen und in einen normalen Arbeitsmodus eingetreten wird, wenn der elektrische Griff die Betriebsanweisung empfängt oder der Adapter den Arbeitskopf in dem Fall identifiziert, dass sich der elektrische Griff im Modus mit geringem Energieverbrauch befindet.

10. Elektrisches Klammergerät, umfassend die Steuerungsvorrichtung mit geringem Energieverbrauch für den Griff des elektrischen Klammergeräts nach einem der Ansprüche 6 bis 9.

## Revendications

1. Procédé de commande à faible consommation d'énergie pour une poignée d'une agrafeuse électrique, l'agrafeuse électrique comprenant une poignée électrique et un adaptateur, la poignée électrique comprenant un compartiment de batterie conçu pour installer un bloc-batterie, et le procédé de commande à faible consommation d'énergie pour la poignée comprenant :
démarrer le comptage de temps en installant le bloc-batterie dans le compartiment de batterie, et détecter simultanément si la poignée électrique reçoit une instruction de fonctionnement et contrôler l'adaptateur pour identifier une tête de travail (S101) ;
si une durée de comptage de temps atteint un premier temps prédéfini, que la poignée électrique ne reçoit pas une instruction de fonctionnement et que l'adaptateur n'identifie pas une tête de travail, commander à un réseau d'alimentation électrique d'entraînement et à un réseau d'alimentation électrique à effet Hall de l'agrafeuse électrique d'arrêter la sortie d'alimentation électrique, et déclencher un contrôleur de poignée (20) pour passer à un état de veille de telle sorte que la poignée électrique passe à un mode à faible consommation d'énergie (S102),
le réseau d'alimentation électrique à effet Hall comprenant : un deuxième tube de commutation (Q2), une cinquième résistance (R5), une deuxième diode (D2), une sixième résistance (R6), un deuxième condensateur (C2), une septième résistance (R7), une huitième résistance (R8) et une deuxième puce de commutation (U2), la sixième résistance (R6) et le deuxième condensateur (C2) étant connectés en parallèle, la septième résistance (R7) et la huitième résistance (R8) étant connectées en série,
commander un réseau d'alimentation électrique d'entraînement et un réseau d'alimentation électrique à effet Hall de l'agrafeuse électrique pour arrêter la sortie d'alimentation électrique comprenant :
l'émission, par le contrôleur de poignée (20), d'un signal de commande à bas niveau vers le réseau d'alimentation électrique d'entraînement et vers le réseau d'alimentation électrique à effet Hall, respectivement,
un terminal de commande du deuxième tube de commutation (Q2) étant conçu pour recevoir le signal de commande à bas niveau, et le deuxième tube de commutation (Q2) et la deuxième puce de commutation (U2) étant dans un état bloqué.

2. Procédé de commande à faible consommation d'énergie pour la poignée de l'agrafeuse électrique selon la revendication 1, la poignée électrique étant en mode à faible consommation d'énergie et le procédé de commande à faible consommation d'énergie comprenant en outre : si la poignée électrique reçoit l'instruction de fonctionnement ou que l'adaptateur identifie la tête de travail, commander à la poignée électrique de sortir du mode à faible consommation d'énergie et de passer à un mode de fonctionnement normal.

3. Procédé de commande à faible consommation d'énergie pour la poignée de l'agrafeuse électrique selon la revendication 1, le premier temps prédéfini étant 30-50 secondes.

4. Support de stockage lisible par ordinateur, dans lequel un programme de commande à faible consommation d'énergie pour une poignée d'une agrafeuse électrique est enregistré, le procédé de commande à faible consommation d'énergie pour la poignée de l'agrafeuse électrique selon l'une quelconque des revendications 1-3 étant effectué par l'exécution du programme de commande à faible consommation d'énergie pour la poignée.

5. Agrafeuse électrique, comprenant :
une poignée électrique, comprenant un compartiment de batterie, le compartiment de batterie étant conçu pour installer un bloc-batterie ; et
un adaptateur,
la poignée électrique comprenant en outre :
une mémoire, conçue pour enregistrer un programme de commande à faible consommation d'énergie pour la poignée
de l'agrafeuse électrique ; et
un processeur, conçu pour exécuter le programme de commande à faible consommation d'énergie pour la poignée, de telle sorte que le procédé de commande à faible consommation d'énergie pour la poignée de l'agrafeuse électrique selon l'une quelconque des revendications 1-4 est effectué.

6. Dispositif de commande à faible consommation d'énergie pour une poignée d'une agrafeuse électrique, l'agrafeuse électrique comprenant une poignée électrique et un adaptateur, la poignée électrique comprenant un compartiment de batterie conçu pour installer un bloc-batterie, le dispositif de commande à faible consommation d'énergie pour la poignée comprenant :
un compteur de temps (10) ; et
un contrôleur de poignée (20) :
le contrôleur de poignée (20) étant conçu pour détecter si la poignée électrique reçoit une instruction de fonctionnement et commande à l'adaptateur d'identifier une tête de travail ;
**caractérisé en ce que**
le contrôleur de poignée (20) est conçu pour déclencher le compteur de temps (10) pour démarrer le comptage de temps lors de l'installation du bloc-batterie dans le compartiment de batterie,
**en ce que** le contrôleur de poignée (20) est conçu en outre pour commander un réseau d'alimentation électrique d'entraînement et un réseau d'alimentation électrique à effet Hall de l'agrafeuse électrique pour arrêter la sortie d'alimentation électrique et passer à un état de veille, si une durée de comptage de temps du compteur de temps (10) atteint un premier temps prédéfini, la poignée électrique ne reçoit pas l'instruction de fonctionnement, et l'adaptateur n'identifie pas la tête de travail, de telle sorte que la poignée électrique passe à un mode à faible consommation d'énergie,
**en ce que** le contrôleur de poignée (20) est conçu en outre pour sortir un signal de commande à bas niveau vers le réseau d'alimentation électrique d'entraînement et vers le réseau d'alimentation électrique à effet Hall, respectivement, afin de commander au réseau d'alimentation électrique d'entraînement et au réseau d'alimentation électrique à effet Hall d'arrêter la sortie d'alimentation électrique,
le réseau d'alimentation électrique à effet Hall comprenant : un deuxième tube de commutation (Q2), une cinquième résistance (R5), une deuxième diode (D2), une sixième résistance (R6), un deuxième condensateur (C2), une septième résistance (R7), une huitième résistance (R8) et une deuxième puce de commutation (U2), la sixième résistance (R6) et le deuxième condensateur (C2) étant connectés en parallèle, la septième résistance (R7) et la huitième résistance (R8) étant connectées en série,
**en ce qu'**un terminal de commande du deuxième tube de commutation (Q2) est conçu pour recevoir le signal de commande à bas niveau, et le deuxième tube de commutation (Q2) et la deuxième puce de commutation (U2) étant dans un état bloqué.

7. Dispositif de commande à faible consommation d'énergie pour la poignée de l'agrafeuse électrique selon la revendication 6, le réseau d'alimentation électrique d'entraînement comprenant :
un premier tube de commutation (Q1), une première résistance (R1), une première diode (D1), une deuxième résistance (R2), un premier condensateur (C1), une troisième résistance (R3), une quatrième résistance (R4), une première puce de commutation (U1) et un tube de stabilisation de tension (Z1), la deuxième résistance (R2) et le premier condensateur (C1) étant connectés en parallèle, la troisième résistance (R3) et la quatrième résistance (R4) étant connectées en série.

8. Dispositif de commande à faible consommation d'énergie pour la poignée de l'agrafeuse électrique selon la revendication 7, un terminal de commande du premier tube de commutation (Q1) étant conçu pour recevoir le signal de commande à faible niveau, et le premier tube de commutation (Q1) et la première puce de commutation (U1) étant dans un état bloqué.

9. Dispositif de commande à faible consommation d'énergie pour la poignée de l'agrafeuse électrique selon la revendication 6, le contrôleur de poignée (20) étant conçu en outre pour commander la poignée électrique pour sortir du mode à faible consommation d'énergie et passer à un mode de travail normal si la poignée électrique reçoit l'instruction de fonctionnement ou si l'adaptateur identifie la tête de travail au cas où la poignée électrique est en mode à faible consommation d'énergie.

10. Agrafeuse électrique, comprenant le dispositif de commande à faible consommation d'énergie pour la poignée de l'agrafeuse électrique selon l'une quelconque des revendications 6-9.
